# EUROPEAN PATENT SPECIFICATION

(11) **EP 4 312 746 B1**
(45) Date of publication and mention of the grant of the patent: **25.12.2024**
(21) Application number: 22712657.0
(22) Date of filing: 23.03.2022
(51) Int. Cl.: A61B 5/08, A61B 5/03, A61B 5/00, A61M 16/06

(54) **APPARATUS FOR DETECTING AND MONITORING NASAL PRESSURE**
GERÄT ZUR ERFASSUNG UND ÜBERWACHUNG DES NASENDRUCKS
APPAREIL POUR DÉTECTER ET SURVEILLER LA PRESSION NASALE

(30) Priority: 26.03.2021 IT 202100007478
(43) Date of publication of application: 07.02.2024
(73) Proprietor: Fantini, Riccardo, 42122 Reggio nell'Emilia (RE) (IT); Tonelli, Roberto, 41122 Modena (MO) (IT); Castaniere, Ivana, 41122 Modena (MO) (IT); Clini, Enrico, 25124 Brescia (BS) (IT); Tabbí, Luca, 41124 Modena (MO) (IT); Marchioni, Alessandro, 41125 Modena (MO) (IT)
(72) Inventor: Fantini, Riccardo, 42122 Reggio nell'Emilia (RE) (IT); Tonelli, Roberto, 41122 Modena (MO) (IT); Castaniere, Ivana, 41122 Modena (MO) (IT); Clini, Enrico, 25124 Brescia (BS) (IT); Tabbí, Luca, 41124 Modena (MO) (IT); Marchioni, Alessandro, 41125 Modena (MO) (IT)
(74) Representative: Corradini, Corrado
(86) International application number: PCT/IB2022/052643
(87) International publication number: WO 2022/201051

(56) References cited:
- EP-B1- 3 421 069
- US-A- 4 273 124
- US-A- 5 477 852
- US-A1- 2008 051 674

## Description

### Technical Field

The present invention relates to devices for measuring the inspiratory pressure of a patient. In particular, the invention relates to a device for measuring a differential of the nasal pressure of a patient between an inhalation phase and an exhalation phase.

### Background Art

Apparatuses for measuring values indicative of a patient's respiratory capacity are known.

These measurement apparatuses have often been used in the past to better understand the dynamics of basic physiological mechanisms and to delineate the physiology of respiratory mechanics.

However, the same apparatuses are still widespread and are also used in clinical practice, for example to monitor mechanical ventilation, spontaneous or assisted breathing of a patient.

In particular, these apparatuses have proved to be very useful in detecting the presence of diaphragmatic paralysis and in making an assessment of the respiratory work during mechanical ventilation.

They are therefore widely used in intensive care units, where monitoring respiratory mechanics by observing the patient's ventilation and breathing pattern is vital. The most commonly used apparatuses in this context are those for measuring oesophageal and gastric pressure, which allow a doctor to determine the values of pleural pressure and abdominal pressure and to analyse the pulmonary and thoracic compliance (distensibility) of the patient under examination.

These apparatuses typically use an oesophageal balloon associated with a catheter, through which the balloon is introduced nasally up to the patient's oesophagus. Once the oesophageal balloon has been introduced, it can be properly inflated, after which pressure measurements can begin.

However, this solution presents some criticalities, in particular regarding the invasiveness of the operation.

In fact, as specified above, apparatuses of this kind are mainly used in intensive care units, where patients are often in a condition of respiratory insufficiency. Because of this condition, patients are often connected to mechanical ventilation, or assisted breathing devices that replace or assist the normal activity of the inspiratory muscles so as to ensure that the lungs receive sufficient oxygen.

These assisted breathing devices normally make use of breathing masks or helmets that are placed on the patient's face.

It follows that the possible introduction of a catheter, possibly provided with an oesophageal balloon, inevitably proves to be a hindrance and can therefore affect the proper operation of these devices, sometimes putting the patient's health at risk.

US2008051674, for example, describes a diagnostic tool for measuring nasal cavity pressure of a patient, during both inhalation and exhalation. The diagnostic tool comprising a the nasal cannula having a pair of supply lines, each of which has a head with a discharge opening for discharging a respiratory gas. Each head is sized to be inserted within one of the nasal cavities of the patient. The tool comprises a pressure sensing probe associated with each head. Each of the pressure sensing probe is coupled to a pressure sensing device to provide a pressure reading.

The diagnostic tool described in this document should only be associated with the patient during measurement tasks because, the introduction of the head, as well as catheters and oesophageal balloons, by nasal or oral route, in order to measure respiratory pressure, is very invasive and often causes great discomfort to the patient.

Experience is required for the proper positioning of the oesophageal tube which limits the widespread use of the procedure in non-intensive settings.

Finally, the device in question is burdened by high costs since it is a singlepatient device that cannot be reused.

### Description of the Invention

The object of the present invention is to solve or at least mitigate the above-mentioned drawbacks, within a simple, rational and relatively low cost solution. This and other objects are reached by the characteristics of the invention as set forth in the independent claims. The dependent claims outline preferred and/or particularly advantageous aspects of the invention.

In particular, an embodiment of the present invention makes available an apparatus for detecting and monitoring nasal pressure comprising:
- a nasal probe adapted to be permanently placed inside a patient's nostril, wherein the nasal probe comprises:
- a support ring which defines inside it a through opening and provided with an external surface adapted to come into contact with the patient's nostril,
- a housing seat defined within the external surface of the support ring for housing the end of a cannula (catheter) adapted to be connected to a pressure transducer, and
- an inflatable membrane (cuff) associated with the support ring and configured to be selectively moved between:
   o a first configuration, in which it is inflated and completely occludes the through opening, and
   ∘ a second configuration, in which it is deflated and leaves at least partially open the through opening.

In this way, the invention is presented as a practical and minimally invasive solution for performing a correct measurement of respiratory pressure.

Thanks to this invention it is in fact possible to measure the respiratory pressure without using any oesophageal catheter and therefore without interfering with any artificial or assisted breathing devices and with less discomfort for the patient, especially during the insertion step.

In particular, the invention offers the significant advantage of making use of a nasal probe to be housed at the level of the nostril and therefore with a low level of invasiveness.

After insertion, this nasal probe can be left in place for a prolonged time, even during assisted ventilation and/or in conjunction with the presence of other therapeutic instruments that need to be positioned on the patient's face.

With the inflatable membrane in a deflated configuration, air will be able to pass freely through the through opening of the support ring, allowing the patient to breathe normally.

Only when it becomes necessary to take the measurement, it is possible to inflate the membrane and obstruct the through opening and then the nostril, allowing the pressure transducer to effectively measure the respiratory pressure through the cannula.

In this way, the invention provides the possibility to perform multiple, continuous measurements of the patient's respiratory effort without interfering with the patient's spontaneous breathing.

This solution is also advantageous in that it has a housing seat that is adapted to accommodate and support the end of the cannula, thus preventing any discomfort to the patient.

The nature of the invention also allows the probe to be easily and quickly removed from the patient's nostril, for example in case of respiratory emergencies and, more generally, if there is a need to use the nostril for other procedures such as bronchoscopies, nasal tracheal intubations or nasogastric tube positionings. In the latter case, the probe can be removed and positioned on the contralateral nostril, so as to ensure the measurement of the patient's breathing capacity.

According to an aspect of the present invention, the support ring of the nasal probe may comprise a first internal annular portion and a second external annular portion of lower stiffness than the first annular portion, which second annular portion externally covers the first annular portion and defines the external surface adapted to come into contact with the patient's nostril.

In this way, the first portion can perform an essentially structural function, necessary to keep the cannula inside the patient's nostril, while the second portion can reduce the discomfort for the patient, adapting at least partially to the shape of the nostril.

By way of example, the first annular portion of the support ring may be made of plastics while the second annular portion may be made of rubber.

Rubber is in fact a sufficiently soft and non-irritating material so as not to cause discomfort to the patient, whereas plastics has the characteristics of stiffness and solidity necessary to offer a valid support to the cannula.

Going into more detail, one aspect of the invention provides that the nasal probe may comprise an eyelet provided with an internal surface and with an external surface, wherein the internal surface of the eyelet defines the housing seat of the cannula, and wherein the through opening is instead delimited between the external surface of said eyelet and an internal surface of the support ring.

This aspect provides a rather simple and rational solution to realize both the through opening and the accommodating seat for the cannula, inside the support ring.

In this context, an embodiment of the invention provides that the eyelet of the nasal probe may be arranged coaxial to the support ring and is connected to the latter by at least one connecting element.

In this way, the cannula remains substantially equidistant from the walls of the nostril, reducing the possibility of creating a discomfort to the patient. According to a different embodiment, the eyelet of the nasal probe can be abutted to the support ring and thus partially integrated therewith.

This allows both the external ring and the eyelet to be made as one compact body, making the nasal probe more solid and stable, as well as making it easier to manufacture.

According to a different aspect of the invention, the inflatable membrane may be fixed to the internal surface of the support ring and may be adapted to expand towards external surface of the eyelet.

In other cases, the inflatable membrane may alternatively be fixed to the external surface of the eyelet and be adapted to expand towards the internal surface of the support ring.

Both of these solutions ensure an effective closure of the through opening, favouring the correct operation of the apparatus and the correct measurement of the pressure values.

These solutions also ensure that the membrane does not impede breathing when deflated.

Another aspect of the invention provides that the apparatus may further comprise a system for inflating and deflating the inflatable membrane, for example by means of an air syringe.

This solution allows a user, such as a doctor, to move the inflatable membrane between the first and the second configuration and vice versa, quickly and easily. According to another aspect of the invention, the apparatus may further comprise a pressure transducer connected with the cannula, which can be configured to measure a pressure difference between an inhalation phase and an exhalation phase.

This pressure transducer will obviously not be housed inside the nasal probe but will be an external instrument, for example one of those pressure transducers normally present in hospitals or possibly a device (monitor) specifically dedicated to the apparatus in question.

Another embodiment of the present invention finally makes available a method for detecting and monitoring nasal pressure of a patient, which comprises the following steps:
- preparing an apparatus according to any one of the preceding claims,
- housing the end of the cannula in the housing seat of the nasal probe,
- connecting said cannula with a pressure transducer,
- positioning the nasal probe inside one of the patient's nostrils,
- inflating the inflatable membrane, by occluding the opening through the nos-tril,
- measuring by means of the pressure transducer at least one pressure difference between an inhalation phase and an exhalation phase,
- deflating the inflatable membrane, reopening at least partially the through opening and thus the nostril.

Taking advantage of the apparatus described above, this method essentially achieves the same advantages, namely those of allowing a measurement of respiratory pressure, without interfering with other respiratory apparatus and with less discomfort for patients.

The simplicity of positioning in an external cavity makes it easier to learn the method in question, making the instrument more distributable even in areas with lower care intensity but which also treat patients with respiratory insufficiency.

### Brief Description of the Drawings

Further characteristics and advantages of the invention will become clear from reading the following description provided by way of non-limiting example, with the aid of the figures illustrated in the accompanying tables.
Figure 1 is a front view of a nasal probe of an apparatus for detecting and monitoring nasal pressure according to the invention.
Figure 2 is a front view of the nasal probe according to a different embodiment of the invention.
Figure 3 is the section III-III of Figure 1.
Figure 4 is a schematic view of the apparatus according to the invention, comprising the section of figure 3.

### Embodiments of the Invention

With particular reference to the aforementioned figures, an apparatus for detecting and monitoring the nasal pressure of a patient has been globally indicated as 10. More specifically, the apparatus 10 in question is configured to obtain a measurement of a differential of nasal pressure between an inhalation phase and an exhalation phase, which is then intended to be analysed in order to provide an indication of the patient's respiratory capacity.

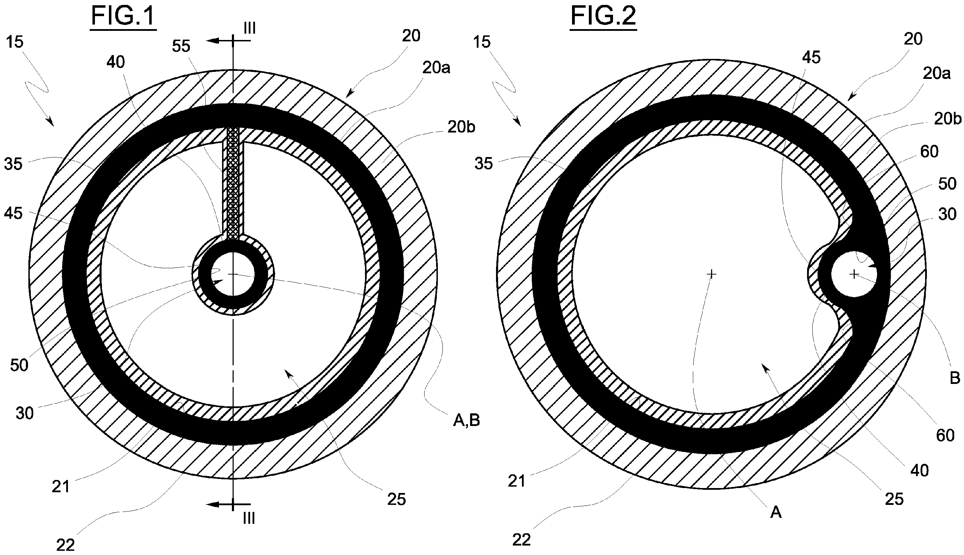

As illustrated in Figure 1, the apparatus 10 comprises first of all a nasal probe 15 adapted to be stably placed within a nostril of the patient.

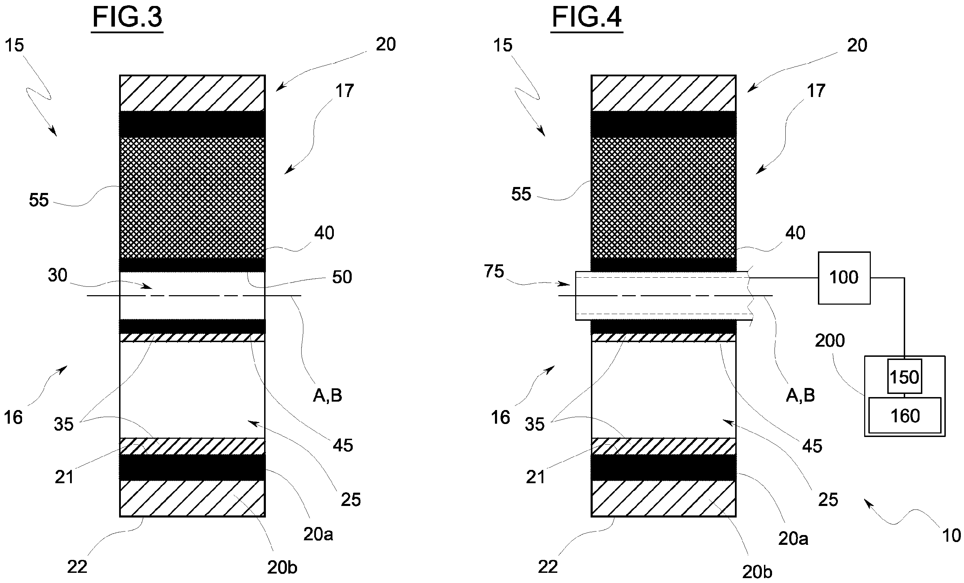

More precisely, the nasal probe 15 is designed to be inserted into the nostril and to stably remain there until it is necessary to remove it, for example due to any operations that require the use of the nostril in which it has been positioned. The nasal probe 15 has a support ring 20, for example a cylindrical shaped body, which develops longitudinally along a central axis A from a first end 16 to a second end 17 (see fig.3) and which is adapted to come into contact with the patient's nostril, when the nasal probe 15 is inserted.

Given the insertion mode of the nasal probe 15, which is designed to be slidingly inserted into the patient's nostril, one of the first end 16 and the second end 17 of the support ring 20 is intended to face inwardly of the nasal cavity, while the other end is intended to face in the opposite direction, i.e., outwardly of the patient's nostril.

The support ring 20 generally comprises an internal surface 21 facing radially towards the central axis A and an external surface 22 facing radially in the opposite direction.

These internal 21 and external 22 surfaces may have a circular cross-section centred on the central axis A.

The radial distance between the internal surface 21 and the central axis A is clearly greater than the distance between the central axis A and the external surface 22, so that between these two surfaces the thickness of the support ring 20 remains defined, which may depend on the size of the patient's nostril. When the nasal probe 15 is inserted into a patient's nostril, the external surface 22 of the support ring 20 is intended to be in contact with the internal surface of the nostril.

Instead, the internal surface 21 defines a through opening 25, i.e., an internal cavity extending along the entire length of the support ring 20, in the direction of the central axis A.

In particular, the through opening 25 extends into the space included between the first end 16 and the second 17 end of the support ring 20.

In this way, despite the presence of the nasal probe 15, the through opening 25 allows flow communication between the inside and the outside of the nostril and, consequently, allows the patient to breathe through that nostril, as well. In other words, the function of the through opening 25 is to allow the patient to breathe through both nostrils, even if one of them is occupied by the nasal probe 15.

Going into more detail, the support ring 20 may comprise a first internal annular portion 20a and a second external annular portion 20b (always with reference to the central axis A).

The first annular portion 20a is at a shorter distance radially from the central axis A than the second annular portion 20b and is therefore externally covered by the latter.

Due to this arrangement, the first annular portion 20a defines the internal surface 21 of the support ring 20, while the second annular portion 20b defines the external surface 22.

Since the external surface 22 is intended to be in contact with the nostril, the second annular portion 20b is preferably made so as to have a lower stiffness than the first annular portion 20a.

For example, the second annular portion 20b may be made of rubber, while the first annular portion 20a may be made of plastics.

In this way, the first annular portion 20a provides sufficient stiffness to ensure structural stability of the nasal probe 15, while the second annular portion 20b is more comfortable for the patient.

In fact, especially during insertion of the nasal probe 15, the material of the second annular portion 20b may be subjected to a slight compression to be adapted to the size and shape of the patient's nostril, so as to better accommodate the nasal probe 15.

The nasal probe 15 further comprises a housing seat 30 defined within the external surface 22 of the support ring 20 and designed to house, through insertion, the end of a tubular cannula (or catheter) 75.

The aforesaid housing seat 30 is of the type passing through, i.e., it is configured as a through hole extending along an axis B parallel to the central axis A and having opposite open ends.

In this way, the housing seat 30 allows the end of the cannula 75 to be supported within the patient's nostril, but without occluding the through opening 25.

The diameter of the housing seat 30 may be equal to or slightly smaller than the diameter of the cannula 75, so as not to hinder the insertion thereof but to be able to stably block it in place.

According to an embodiment illustrated in figure 1, the housing seat 30 is defined by an eyelet 40, which is placed internally to the internal surface 21 of the support ring 20.

The aforesaid eyelet 40 is provided with an external surface 45 and with an internal surface 50 (with respect to the central axis A).

The internal surface 50 of the eyelet 40 defines the housing seat 30 of the cannula 75, whereas the space included between the internal surface 21 of the support ring 20 and the external surface 45 of the eyelet 40 defines the through opening 25. In particular, the eyelet 40 may be coaxially arranged within the support ring 20 such that the central axis A of the latter coincides with the axis B of the housing seat 30.

In this particular conformation, the eyelet 40 can be securely fixed to the support ring 20, or more precisely to the first annular portion 20a, by means of a connecting element 55.

For example, the first annular portion 20a of the support ring 20, the eyelet 40, and the connecting member 55 may be made as a single body, that is, as a single monolithic body.

According to an alternative embodiment illustrated in Figure 2, the eyelet 40 may be abutted to and partially integrated with the support ring 20, for example the first annular portion 20a.

In particular, the eyelet 40 may present itself as being part of the support ring 20, or more precisely of the first annular portion 20a, being at least partially protruding from the internal surface 21, radially towards the central axis A.

Also in this case, the housing seat 30 remains defined by the internal surface 50 of the eyelet 40, which is always contained within the external surface 22 of the support ring 20.

On the other hand, the external surface 45 of the eyelet 40 is coupled to the internal surface 21 of the support ring 20, with which it substantially forms a single surface adapted to delimit the through opening 25.

In both cases, the support ring 20 further comprises an inflatable membrane 35, which is adapted to be internally associated with the support ring 20.

Said inflatable membrane 35 is configured to be selectively moved between a first configuration and a second configuration.

In the first configuration (illustrated in the figures), the inflatable membrane 35 is deflated and leaves at least partially open the through opening 25.

More precisely, in the first configuration, the inflatable membrane 35 does not completely occlude the through opening 25 and leaves the nostril in communication with the external environment, thus allowing normal breathing to the patient, even through the nasal probe 15.

In the second configuration (not illustrated), the inflatable membrane 35 is inflated and completely occludes the through opening 25 of the support ring 20, preventing breathing from the nostril housing the nasal probe 15.

In particular, in the embodiments disclosed herein, the inflatable membrane 35 may be fixed to the internal surface 21 of the support ring 20, for example by using an adhesive material.

In this way, when the inflatable membrane 35 is deflated, a portion of the through opening 25 remains clear.

During inflation, the inflatable membrane 35 expands until it completely covers the external surface 45 of the eyelet 40, by completely occluding the through opening 25 of the nasal probe 15.

In other embodiments (not illustrated), the inflatable membrane 35 could alternatively be fixed to the external surface 45 of the eyelet 40 so as to expand during inflation until it completely covers the internal surface 21 of the support ring 20. In all cases, the apparatus 10 may comprise an inflation and deflation system (not shown), for example but not necessarily an air syringe, by which inflation of the inflatable membrane 35 and deflation thereof following the measurement of the patient's nasal pressure is performed.

As illustrated in Figure 4, the apparatus 10 further comprises a pressure transducer 100 connected with the cannula 75, which may be configured to measure a pressure differential between an inhalation phase and an exhalation phase of the patient.

Of course the pressure transducer 100 is not housed within the nasal probe 15 but is an external instrument.

It may, for example, be a pressure transducer of those normally present in hospitals or a device (monitor) expressly dedicated to the apparatus 10.

The cannula 75 is shaped like a tube possibly flexible and having two opposite open ends.

A first of these ends is coupled to the housing seat 30 of the nasal probe 15 (as specified above) while the second end is connected with the pressure transducer 100, so that the latter can detect the same pressure that reigns in the patient's nostril.

The pressure transducer 100 is then configured to continuously convert the nasal pressure, detected through the cannula 75, into an analogue electrical signal. The apparatus 10 may therefore comprise an analogue-to-digital converter 150, which is operatively connected to the pressure transducer 100 and adapted to convert the analogue electrical signal into a discrete (digital) signal.

More specifically, the analogue-to-digital converter 150 may be configured to convert the analogue electrical signal indicative of the patient's nasal pressure into a discrete value indicative of the same pressure and/or another parameter of the patient's respiratory capacity.

In other cases, the pressure transducer 100 may be a digital type and thus directly provide a digital signal.

Additionally, the apparatus 10 may comprise a monitor 160, operatively connected to the analogue-to-digital converter 150 or directly to the pressure transducer 100, which is configured to continuously display the pressure and/or respiratory capacity value of the patient.

The monitor 160 and/or the analogue-to-digital converter 150 may be integrated with or connected to a computer 200, which is configured to process and manage data produced by the pressure transducer 100.

In use, the apparatus 10 is first of all prepared by inserting one end of the cannula 75 into the housing seat 30 of the nasal probe 15 and by connecting the opposite end to the pressure transducer 100.

The nasal probe 15 is then housed within one of the patient's nostrils, where it remains until use thereof is over.

When it is necessary to measure the respiratory pressure, the inflatable membrane 35 is inflated so that it expands until it completely occludes the through opening 25 of the nasal probe 15, and consequently also the nostril.

At this point, the pressure transducer 100 can measure, through the cannula 75 the nasal pressure values during at least one exhalation phase and at least one inhalation phase of the patient, from which a pressure differential between the two phases can be derived and/or calculated.

At the end of the measurement, the inflatable membrane 35 can be deflated so that the patient can breathe with both nostrils.

At this point, the nasal probe 15 can be held within the nostril so that the measurement can be repeated later.

If or when it is deemed that repeating the measurement is no longer necessary, the nasal probe 15 may be removed.

The invention thus conceived is susceptible to several modifications and variations, all falling within the scope of the inventive concept. Moreover, all details can be replaced by other technically equivalent elements. In practice, the materials used, as well as the contingent shapes and sizes, can be whatever according to the requirements without for this reason departing from the scope of protection of the following claims.

## Claims

1. An apparatus (10) for detecting and monitoring nasal pressure comprising:
- a nasal probe (15) adapted to be permanently placed inside a patient's nostril, wherein the nasal probe (15) comprises:
- a support ring (20) which defines inside it a through opening (25) and provided with an external surface (22) adapted to come into contact with the patient's nostril, and
- a housing seat (30) defined within the external surface (22) of the support ring (20) for housing the end of a cannula (75) adapted to be connected to a pressure transducer (100),
**characterized by** the fact that it comprises
an inflatable membrane (35) associated with the support ring (20) and configured to be selectively moved between:
- a first configuration, in which it is inflated and completely occludes the through opening (25), and
- a second configuration, in which it is deflated and leaves at least partially open the through opening (25).

2. Apparatus (10) according to claim 1, wherein the support ring (20) of the nasal probe (15) comprises a first internal annular portion (20a) and a second external annular portion (20b) of lower stiffness than the first annular portion, which second annular portion (20b) externally covers the first annular portion (20a) and defines the external surface (22) of the support ring (20) adapted to come into contact with the patient's nostril.

3. Apparatus (10) according to claim 2, wherein the first annular portion (20a) of the support ring (20) is made of plastics and the second annular portion (20b) is made of rubber.

4. Apparatus (10) according to any one of the preceding claims, wherein the nasal probe (15) comprises an eyelet (40) provided with an internal surface (50) and with an external surface (45), wherein the internal surface (50) of the eyelet (40) defines the housing seat (30) of the cannula (75), and wherein the through opening (25) is delimited between the external surface (45) of the eyelet (40) and an internal surface (21) of the support ring (20).

5. Apparatus (10) according to claim 4, wherein the eyelet (40) of the nasal probe (15) is arranged coaxial to the support ring (20) and is connected thereto by at least one connecting element (55).

6. Apparatus (10) according to claim 4, wherein the eyelet (40) of the nasal probe is abutted to and partially integrated with the support ring (20).

7. Apparatus (10) according to claim 4, wherein the inflatable membrane (35) is fixed to the internal surface (21) of the support ring and is adapted to expand towards the external surface (45) of the eyelet (40).

8. Apparatus (10) according to claim 4, wherein the inflatable membrane (35) is fixed to the external surface (45) of the eyelet (40) and is adapted to expand towards the internal surface (21) of the support ring (20).

9. Apparatus (10) according to any one of the preceding claims, comprising a system for inflating and deflating the inflatable membrane (35).

10. Apparatus (100) according to claim 9, comprising a pressure transducer connected with the cannula (75).

11. Method for detecting and monitoring the nasal pressure of a patient comprising the following steps:
- preparing an apparatus (10) according to any one of the preceding claims,
- housing one end of the cannula (75) in the housing seat (30) of the nasal probe (15),
- connecting said cannula (75) with a pressure transducer (100),
- positioning the nasal probe (15) inside one of the patient's nostrils,
- inflating the inflatable membrane (35), by occluding the opening (25) and then the nostril,
- measuring by means of the pressure transducer (100) at least one pressure difference between an inhalation phase and an exhalation phase,
- deflating the inflatable membrane (35), releasing at least part of the opening (25) and then the nostril.

## Patentansprüche

1. Vorrichtung (10) zum Erfassen und Überwachen von Nasendruck, umfassend:
- eine Nasensonde (15), die angepasst ist, um dauerhaft in dem Nasenloch eines Patienten platziert zu werden, wobei die Nasensonde (15) Folgendes umfasst:
- einen Stützring (20), der in seinem Inneren eine Durchgangsöffnung (25) definiert und mit einer Außenfläche (22) versehen ist, die angepasst ist, um mit dem Nasenloch des Patienten in Kontakt zu kommen, und
- einen Gehäusesitz (30), der innerhalb der Außenfläche (22) des Stützrings (20) definiert ist, um das Ende einer Kanüle (75) aufzunehmen, die angepasst ist, um mit einem Druckmesswertgeber (100) verbunden zu werden,
**dadurch gekennzeichnet, dass** sie Folgendes umfasst
- eine aufblasbare Membran (35), die mit dem Stützring (20) assoziiert und konfiguriert ist, um selektiv bewegt zu werden zwischen:
- einer ersten Konfiguration, in der sie aufgeblasen ist und die Durchgangsöffnung (25) vollständig verschließt, und
- einer zweiten Konfiguration, in der sie entleert ist und die Durchgangsöffnung (25) zumindest teilweise offen lässt.

2. Vorrichtung (10) nach Anspruch 1, wobei der Stützring (20) der Nasensonde (15) einen ersten inneren ringförmigen Abschnitt (20a) und einen zweiten äußeren ringförmigen Abschnitt (20b) mit geringerer Steifigkeit als der erste ringförmige Abschnitt umfasst, wobei der zweite ringförmige Abschnitt (20b) den ersten ringförmigen Abschnitt (20a) von außen bedeckt und die Außenfläche (22) des Stützrings (20) definiert, die angepasst ist, um mit dem Nasenloch des Patienten in Kontakt zu kommen.

3. Vorrichtung (10) nach Anspruch 2, wobei der erste ringförmige Abschnitt (20a) des Stützrings (20) aus Kunststoff gefertigt ist und der zweite ringförmige Abschnitt (20b) aus Gummi gefertigt ist.

4. Vorrichtung (10) nach einem der vorherigen Ansprüche, wobei die Nasensonde (15) eine Öse (40) umfasst, die mit einer Innenfläche (50) und einer Außenfläche (45) versehen ist, wobei die Innenfläche (50) der Öse (40) den Gehäusesitz (30) der Kanüle (75) definiert, und wobei die Durchgangsöffnung (25) zwischen der Außenfläche (45) der Öse (40) und einer Innenfläche (21) des Stützrings (20) begrenzt ist.

5. Vorrichtung (10) nach Anspruch 4, wobei die Öse (40) der Nasensonde (15) koaxial zu dem Stützring (20) angeordnet und durch mindestens ein Verbindungselement (55) daran verbunden ist.

6. Vorrichtung (10) nach Anspruch 4, wobei die Öse (40) der Nasensonde an dem Stützring (20) anliegt und teilweise darin integriert ist.

7. Vorrichtung (10) nach Anspruch 4, wobei die aufblasbare Membran (35) an der Innenfläche (21) des Stützrings befestigt ist und sich zu der Außenfläche (45) der Öse (40) hin ausdehnen kann.

8. Vorrichtung (10) nach Anspruch 4, wobei die aufblasbare Membran (35) an der Außenfläche (45) der Öse (40) befestigt ist und sich zu der Innenfläche (21) des Stützrings (20) hin ausdehnen kann.

9. Vorrichtung (10) nach einem der vorherigen Ansprüche, umfassend ein System zum Aufblasen und Entleeren der aufblasbaren Membran (35).

10. Vorrichtung (100) nach Anspruch 9, umfassend einen Druckmesswertgeber, der mit der Kanüle (75) verbunden ist.

11. Verfahren zum Erfassen und Überwachen des Nasendrucks eines Patienten, umfassend die folgenden Schritte:
- Herstellen einer Vorrichtung (10) nach einem der vorherigen Ansprüche,
- Aufnehmen eines Endes der Kanüle (75) in den Gehäusesitz (30) der Nasensonde (15),
- Verbinden der Kanüle (75) mit einem Druckmesswertgeber (100),
- Positionieren der Nasensonde (15) in einem der Nasenlöcher des Patienten,
- Aufblasen der aufblasbaren Membran (35) durch Verschließen der Öffnung (25) und dann des Nasenlochs,
- Messen, mittels des Druckmesswertgebers (100), mindestens einer Druckdifferenz zwischen einer Einatmungsphase und einer Ausatmungsphase,
- Entleeren der aufblasbaren Membran (35), wodurch zumindest ein Teil der Öffnung (25) und dann das Nasenloch freigegeben werden.

## Revendications

1. Appareil (10) de détection et de surveillance de la pression nasale comprenant :
- une sonde nasale (15) adaptée pour être placée de façon permanente dans la narine d'un patient, dans lequel la sonde nasale (15) comprend :
- un anneau de support (20) définissant à l'intérieur une ouverture débouchante (25) et pourvu d'une surface externe (22) adaptée pour entrer en contact avec la narine du patient, et
- un siège de logement (30) défini à l'intérieur de la surface externe (22) de l'anneau de support (20) permettant de loger l'extrémité d'une canule (75) adaptée pour être connectée à un transducteur de pression (100),
**caractérisé en ce qu'**il comprend une membrane gonflable (35) associée à l'anneau de support (20) et configuré pour être déplacé de manière sélective entre :
- une première configuration, dans laquelle il est gonflé et obstrue complètement l'ouverture débouchante (25), et
- une deuxième configuration, dans laquelle il est dégonflé et laisse au moins en partie ouvert l'ouverture débouchante (25).

2. Appareil (10) selon la revendication 1, dans lequel l'anneau de support (20) de la sonde nasale (15) comprend une première partie annuaire interne (20a) et une deuxième partie annulaire externe (20b) ayant une rigidité inférieure à la première partie annulaire, laquelle deuxième partie annulaire (20b) recouvre extérieurement la première partie annulaire (20a) et définit la surface externe (22) de l'anneau de support (20) adaptée pour entrer en contact avec la narine du patient.

3. Appareil (10) selon la revendication 2, dans lequel la première partie annulaire (20a) de l'anneau de support (20) est en plastique et la deuxième partie annulaire (20b) est en caoutchouc.

4. Appareil (10) selon l'une quelconque des revendications précédentes, dans lequel la sonde nasale (15) comprend un oeillet (40) comportant une surface interne (50) et une surface externe (45), dans lequel la surface interne (50) de l'oeillet (40) définit le siège de logement (30) de la canule (75), et dans lequel l'ouverture débouchante (25) est délimitée entre la surface externe (45) de l'oeillet (40) et une surface interne (21) de l'anneau de support (20).

5. Appareil (10) selon la revendication 4, dans lequel l'oeillet (40) de la sonde nasale (15) est disposé de manière coaxiale à l'anneau de support (20) et y est relié par au moins un élément de connexion (55).

6. Appareil (10) selon la revendication 4, dans lequel l'oeillet (40) de la sonde nasale est fixé à l'anneau de support (20) et partiellement intégré à celui-ci.

7. Appareil (10) selon la revendication 4, dans lequel la membrane gonflable (35) est fixée à la surface interne (21) de l'anneau de support et est adaptée pour se dilater vers la surface externe (45) de l'œillet (40).

8. Appareil (10) selon la revendication 4, dans lequel la membrane gonflable (35) est fixée à la surface interne (45) de l'oeillet (40) et est adaptée pour se dilater vers la surface interne (21) de l'anneau de support (20).

9. Appareil (10) selon l'une quelconque des revendications précédentes, comprenant un système de gonflage et de dégonflage de la membrane gonflable (35).

10. Appareil (100) selon la revendication 9, comprenant un transducteur de pression connecté à la canule (75).

11. Procédé de détection et de surveillance de la pression nasale d'un patient comprenant les étapes suivantes :
- la préparation d'un appareil (10) selon l'une quelconque des revendications précédentes,
- le logement d'une extrémité de la canule (75) dans le siège de logement (30) de la sonde nasale (15),
- la connexion de ladite canule (75) avec un transducteur de pression (100),
- le positionnement de la sonde nasale (15) à l'intérieur de l'une des narines du patient,
- le gonflage de la membrane gonflable (35), par occlusion de l'ouverture (25) puis de la narine,
- la mesure, au moyen du transducteur de pression (100), d'au moins une différence de pression entre une phase d'inhalation et une phase d'exhalation,
- le dégonflage de la membrane gonflable (35), ce qui libère au moins une partie de l'ouverture (25) puis de la narine.
